# EUROPÄISCHE PATENTSCHRIFT

(11) **EP 1 146 821 B1**
(45) Veröffentlichungstag und Bekanntmachung des Hinweises auf die Patenterteilung: **05.11.2003**
(21) Anmeldenummer: 00902636.0
(22) Anmeldetag: 27.01.2000
(51) Int. Cl.: A61B 17/02, A61F 2/44, A61F 2/46

(54) **CHIRURGISCHES INSTRUMENT ZUM EINFÜHREN VON ZWISCHENWIRBELIMPLANTATEN**
SURGICAL INSTRUMENT FOR INSERTING INTERVERTEBRAL IMPLANTS
INSTRUMENT CHIRURGICAL POUR L'INTRODUCTION D'IMPLANTS INTERVERTEBRAUX

(30) Priorität: 30.01.1999 DE 19903762
(43) Veröffentlichungstag der Anmeldung: 24.10.2001
(73) Patentinhaber: Aesculap AG & Co. KG, 78532 Tuttlingen (DE)
(72) Erfinder: FUSS, Franz, Konstantin, A-2700 Wiener Neustadt (AT); SABITZER, Ronald, J., A-1160 Wien (AT); ECKHOF, Stephan, D-78532 Tuttlingen (DE)
(74) Vertreter: Böhme, Ulrich, Dr. Dipl.-Phys.
(86) Internationale Anmeldenummer: EP0000625
(87) Internationale Veröffentlichungsnummer: WO00044288

(56) Entgegenhaltungen:
- EP-A- 0 641 547
- DE-U- 29 720 022
- DE-U- 29 901 611
- US-A- 5 431 658
- US-A- 5 720 751

## Beschreibung

Die Erfindung betrifft ein chirurgisches Instrument zum Einführen von Zwischenwirbelimplantaten in den Zwischenwirbelraum zwischen benachbarten Wirbelkörpern mit zwei einander gegenüberliegenden Führungskörpern mit einer zum jeweils anderen Führungskörper gerichteten Führung, die gemeinsam eine Führungsbahn zwischen sich ausbilden, längs der ein Zwischenwirbelimplantat seitlich in den Zwischenwirbelraum einschiebbar ist.

Zwischenwirbelimplantate werden anstelle einer entfernten Bandscheibe in den Zwischenwirbelraum zwischen zwei benachbarten Wirbelkörpern eingesetzt, um deren Abstand aufrechtzuerhalten und um zu ermöglichen, daß sich die beiden benachbarten Wirbelkörper nach der Entfernung der Bandscheibe durch eine knöcherne Verbindung stabilisieren.

Die Einführung eines solchen in der Regel plattenförmigen oder käfigförmigen Implantats in den Zwischenwirbelraum kann schwierig sein, da die Wirbelkörper nach der Entfernung der Bandscheibe durch die angreifenden Muskeln gegeneinander gepreßt werden. Es ist daher notwendig, durch geeignete Stabilisierungsvorrichtungen, beispielsweise Knochenplatten mit Knochenschrauben, den Abstand der Wirbelkörper zu fixieren.

Zwischenwirbelimplantate werden üblicherweise zwischen die benachbarten Wirbelkörperflächen eingeschoben, die sich ventral des Wirbelkanals befinden, und daher ist es üblich, das Einsetzen derartiger Zwischenwirbelimplantate von ventral aus durchzuführen. Eine dorsale Einführung ist mit großen Schwierigkeiten verbunden.

Es sind Zwischenwirbelimplantate bekannt, die von dorsal-lateral in den Zwischenwirbelraum eingeführt werden können, ein solcher Wirbelkörper ist beispielsweise in der DE 297 20 022 U1 beschrieben. Dabei ist aber offengelassen, wie es gelingen soll, bei diesem komplizierten Zugang das Zwischenwirbelimplantat in der richtigen Position in den Zwischenwirbelraum einzuführen.

In der EP-A-0 641 547 ist ein Instrument beschrieben, mit dem ein Zwischenwirbelimplantat in den Zwischenwirbelraum eingeschoben werden kann. Dieses Instrument bildet eine Führung mit zwei einander gegenüberliegenden Führungskörpern aus, die gemeinsam eine Führungsbahn ausbilden. In dieser Führungsbahn kann das Zwischenwirbelimplantat bis in den Zwischenwirbelraum verschoben werden.

Um dieses Instrument für Zwischenwirbelimplantate verschiedener Größe verwendbar zu machen, kann vorgesehen werden, daß in Längsnuten der Führungskörper verschiedene Einsätze eingeschoben werden. Dies ist relativ kompliziert und macht außerdem die Verwendung von zusätzlichen Teilen notwendig, die in ihren Abmessungen an die jeweils verwendeten Implantate angepaßt werden müssen.

Es ist Aufgabe der Erfindung, ein gattungsgemäßes chirurgisches Instrument so auszubilden, daß mittels der Führüngskörper auch die Wirbelkörper aufspreizbar sind.

Diese Aufgabe wird bei einem chirurgischen Instrument der eingangs beschriebenen Art erfindungsgemäß dadurch gelöst, daß die Führungskörper in ihrem gegenseitigen Abstand verstellbar sind. Damit ist es möglich, mittels der Führungskörper, die an ihrem freien Ende an den benachbarten Wirbelkörpern anliegen, auch die Wirbelkörper aufzuspreizen, um so den Zugang zu dem Zwischenwirbelraum zu ermöglichen. Die Führungskörper übernehmen somit eine doppelte Aufgabe, nämlich einmal die Aufgabe der Aufspreizung des Zwischenwirbelraums und zum anderen die Aufgabe der Führung des Zwischenwirbelimplantates bis in den Zwischenwirbelraum hinein. Ein solches Instrument wird also mit den beiden Führungskörpern durch den Körperzugang bis in den Zwischenwirbelraum geführt, so daß das freie Ende der Führungskörper in den Zwischenwirbelraum mündet. Ein in den Zwischenwirbelraum einzusetzendes Zwischenwirbelimplantat kann dann längs der in dieser Weise ausgebildeten Führungsbahn vorgeschoben werden, bis das Zwischenwirbelimplantat seitlich in den Zwischenwirbelraum gelangt und dort allein durch das Vorschiebern längs der Führungsbahn die gewünschte Position einnimmt.

Günstig ist es dabei, wenn die Führungsbahn in der Verschiebeebene bogenförmig verläuft, so daß das Implantat am Beginn der Führungsbahn schräg in den Körper eingeführt wird und dann genau quer in den Zwischenwirbelraum gelangen kann.

Bei einer besonders bevorzugten Ausführungsform ist vorgesehen, daß der Zwischenraum zwischen den Führungskörpern zumindest einseitig längs der Führungsbahn offen ist. Es ist daher möglich, das Zwischenwirbelimplantat mittels eines Vorschubinstruments längs der Führungsbahn vorzuschieben, wobei das Vorschubinstrument durch den Zwischenraum zwischen den Führungskörpern hindurchtritt.

Weiterhin kann vorgesehen sein, daß die Führungskörper ein freies Ende aufweisen und dort Verlängerungen tragen, die an den den Zwischenwirbelraum bildenden Wirbelkörpern anlegbar sind und die so neben der Führungsbahn angeordnet sind, daß das längs der Führungsbahn vorgeschobene Zwischenwirbelimplantat am Ende der Führungsbahn neben der Verlängerung an die benachbarten Wirbelkörper anlegbar ist. Die Verlängerungen positionieren also die Führungskörper an den den Zwischenwirbelraum bildenden Wirbelkörpern, befinden sich aber nicht in der Führungsbahn des Zwischenwirbelimplantats, so daß dieses beim Vorschieben längs der Führungsbahn am Ende der Führungsbahn neben diesen Verlängerungen liegt und somit die Führungsbahn verlassen und sich unmittelbar an die benachbarten Wirbelkörper anlegen kann.

Insbesondere können die Verlängerungen als paarweise in Verschieberichtung vom Ende der Führungsbahn in dessen Fortsetzung vorstehende Zinken ausgebildet sein.

Insbesondere kann die Führungsbahn an der Oberseite und an der Unterseite durch zwei Führungsflächen begrenzt werden und an den Seiten durch Seitenwände, die einen schlitzförmigen Zwischenraum zwischen sich einhalten, so daß ein zwischen den Führungskörpern geführtes Zwischenwirbelimplantat in der Führungsbahn auch dann geführt ist, wenn der Abstand der Führungskörper sich vergrößert.

Eine besonders günstige Ausführungsform ergibt sich, wenn die Führungskörper an ihrem dem freien Ende gegenüberliegenden Ende schwenkbar miteinander verbunden sind, ein Aufschwenken der Führungskörper führt dann gleichzeitig zum Aufspreizen der Wirbelkörper im Bereich des Zwischenwirbelraums.

Bei einer besonderen Ausführungsform ist vorgesehen, daß jeder Führungskörper eine ebene Anlagefläche für das Zwischenwirbelimplantat und diese seitlich begrenzende, parallel zueinander längs des Führungskörpers verlaufende Wände aufweist, die in Richtung auf den anderen Führungskörper über die Anlagefläche vorstehen. Die Führungsbahn wird also durch zwei Führungskörper gebildet, die im Querschnitt U-förmig ausgebildet sind und das Zwischenwirbelimplantat an der Oberseite und der Unterseite und teilweise an den Seitenflächen umgeben.

Vorteilhaft ist es, wenn die Führungskörper ein freies Ende aufweisen und wenn die Führungsbahn an ihrem dem freien Ende der Führungskörper gegenüberliegenden Ende seitlich aus dem Instrument austritt, so daß dort ein Zwischenwirbelimplantat in die Führungsbahn einschiebbar ist.

Bei einer besonders bevorzugten Ausführungsform ist vorgesehen, daß zwischen den beiden Führungskörpern längs der von diesen gebildeten Führungsbahn ein Vorschubkörper verschiebbar gelagert ist, der eine lösbare Halterung für das Zwischenwirbelimplantat aufweist. Bei dieser Ausführungsform wird also ein schlittenartiger Führungskörper auf der Führungsbahn als Mitnehmer für das Zwischenwirbelimplantat ausgebildet.

Es ist dabei vorteilhaft, wenn der Vorschubkörper einen zu seinem in Vorschubrichtung vorderen Ende hin offenen Aufnahmeraum für das Zwischenwirbelimplantat aufweist. Dieses wird in diesem Aufnahmeraum aufgenommen, in dieser Anordnung zusammen mit dem Vorschubkörper längs der Führungsbahn vorgeschoben und dann durch Zurückziehen des Vorschubkörpers durch die offene Seite des Aufnahmeraumes wieder aus diesem freigegeben.

Der Aufnahmeraum kann vorzugsweise durch zwei längs der Führungsbahn verlaufende Seitenwände begrenzt werden.

Insbesondere kann vorgesehen sein, daß die lösbare Halterung elastische Rasten umfaßt, die in Rücksprünge einfedern. Das Zwischenwirbelimplantat wird also im Aufnahmeraum durch eine Schnapp- oder Rastverbindung gehalten, die durch kräftiges Herausziehen des Zwischenwirbelimplantates aus dem Aufnahmeraum wieder gelöst werden kann.

Der Vorschubkörper kann an seinem in Vorschubrichtung hinteren Ende eine Zugangsöffnung für ein an dem Zwischenwirbelimplantat anlegbares Rückhalteelement aufweisen, so daß nach dem Einsetzen des Zwischenwirbelimplantates dieses durch das Rückhalteelement in der erreichten Stellung zwischen den Wirbelkörpern zurückgehalten werden kann, wenn der Vorschubkörper wieder zurückgezogen wird. Dabei tritt das Zwischenwirbelimplantat aus dem Aufnahmeraum des Vorschubkörpers aus.

Insbesondere kann das Rückhalteelement entsprechend der Führungsbahn gebogen sein.

Bei einer bevorzugten Ausführungsform ist vorgesehen, daß der Vorschubkörper mit einer längs der Führungsbahn vorschiebbaren, gebogenen Vorschubstange verbunden ist. Die Verbindung zwischen Vorschubkörper und Vorschubstange kann dabei lösbar sein.

Besonders vorteilhaft ist es, wenn die Vorschubstange als Zahnstange ausgebildet ist, welche mit einem an einem Führungskörper drehbar gelagerten Zahnrad kämmt. Durch Verdrehung des Zahnrades kann der Operateur somit die Vorschubstange und damit den Vorschubkörper und das daran gehaltene Zwischenwirbelimplantat längs der Führungsbahn verschieben.

Die Vorschubstange kann eine Längsnut zur Aufnahme und Führung eines Rückhalteelementes für das Zwischenwirbelimplantat aufweisen.

Bei einer besonders bevorzugten Ausführungsform ist vorgesehen, daß die Führungskörper von ihrer Schwenklagerstelle bis zu ihrem freien Ende hin einen abnehmenden Abstand zueinander aufweisen, der an der in Vorschubrichtung hinten angeordneten Einschubseite der Führungsbahn größer ist als die Höhe des Zwischenwirbelimplantates und gegebenenfalls die Höhe des Vorschubkörpers, an der in Vorschubrichtung vorne liegenden Austrittsseite der Führungsbahn jedoch kleiner als die Höhe des Zwischenwirbelimplantates und/oder des Vorschubkörpers. Dadurch wirken der Vorschubkörper und/oder das Zwischenwirbelimplantat beim Vorschieben längs der Führungsbahn als Spreizkörper, die die beiden Führungskörper auseinanderschwenken und dadurch den Abstand zwischen den beiden Wirbelkörpern vergrößern, zwischen die das freie Ende der Führungskörper eingeschoben ist und zwischen die das Zwischenwirbelimplantat eingeschoben werden soll. Die Vorschubbewegung selbst führt also zur Aufspreizung des Zwischenwirbelraumes, der Operateur muß nicht gleichzeitig die Aufspreizung vornehmen und den Vorschub längs der Führungsbahn kontrollieren, sondern es genügt für den Operateur, wenn er das Zwischenwirbelimplantat längs der Vorschubbahn vorschiebt, der für die Einfuhr des Zwischenwirbelimplantates notwendige Abstand der benachbarten Wirbelkörper stellt sich dann zwangsläufig ein.

Günstig ist es dabei, wenn der Vorschubkörper und/oder die Führungskörper an den aneinander anliegenden Flächen aus einem reibungsarmen Material bestehen, beispielsweise können die entsprechenden Flächen mit einem gleitfähigen Kunststoff beschichtet sein.

Bei einer anderen Ausführungsform der Erfindung ist vorgesehen, daß beide Führungskörper mit Griffbranchen fest verbunden sind, die sich über die gelenkige Verbindungsstelle der Führungskörper hinaus erstreckt. Man erhält so ein zangenartiges Instrument, bei dem durch Gegeneinanderdrücken der Griffbranchen die Führungskörper auseinandergeschwenkt werden.

Günstig ist es, wenn an dem Instrument ein Anschlag zur Einhaltung eines Mindestabstands zwischen den beiden Führungskörpern angeordnet ist, dadurch wird sichergestellt, daß sich das Zwischenwirbelimplantat längs seiner Führungsbahn nicht verklemmen kann.

Der Anschlag kann vorzugsweise verstellbar sein, so daß der Mindestabstand an die Größe des jeweiligen Implantats angepaßt werden kann.

Die nachfolgende Beschreibung bevorzugter Ausführungsformen der Erfindung dient im Zusammenhang mit der Zeichnung der näheren Erläuterung. Es zeigen:
- Figur 1:: Eine Draufsicht auf ein chirurgisches Führungs- und Spreizinstrument mit längs der Führungsbahn mittels eines Einsetzinstruments vorgeschobenem Zwischenwirbelimplantat;
- Figur 2:: eine Seitenansicht in Richtung des Pfeils A in Figur 1;
- Figur 3:: eine perspektivische Ansicht des in den Zwischenwirbelraums eingesetzten chirurgischen Instruments mit dem Zwischenwirbelimplantat in der Endposition im Zwischenwirbelraum;
- Figur 4:: eine perspektivische Ansicht eines Zwischenwirbelimplantats im Zwischenwirbelraum mit eingesetzter Vorrichtung zum Einfüllen von Knochenmaterial;
- Figur 5:: eine Draufsicht auf ein erstes bevorzugtes Ausführungsbeispiel eines Zwischenwirbelimplantats mit gebogenen Längsseiten am Ende des chirurgischen Einführinstruments;
- Figur 6:: eine Ansicht ähnlich Figur 5 mit zwei nebeneinander eingesetzten Zwischenwirbelimplantaten am Ende des chirurgischen Einführelements;
- Figur 7:: eine Ansicht ähnlich Figur 6 bei Zwischenwirbelimplantaten mit rechteckigem Querschnitt;
- Figur 8:: eine Draufsicht auf eine weitere bevorzugte Ausführungsform eines chirurgischen Führungs- und Spreizinstrumentes bei abgenommenem oberen Führungskörper;
- Figur 9:: eine perspektivische Ansicht des Instruments der Figur 8 mit abgenommenem oberen Führungskörper;
- Figur 10:: eine Ansicht ähnlich Figur 9 mit aufgesetztem oberen Führungskörper und eingeschobenem Rückhalteelement und
- Figur 11:: eine Ansicht ähnlich Figur 8 mit vollständig eingeschobenem Vorschubkörper und vollständig eingeschobenem Rückhalteelement.

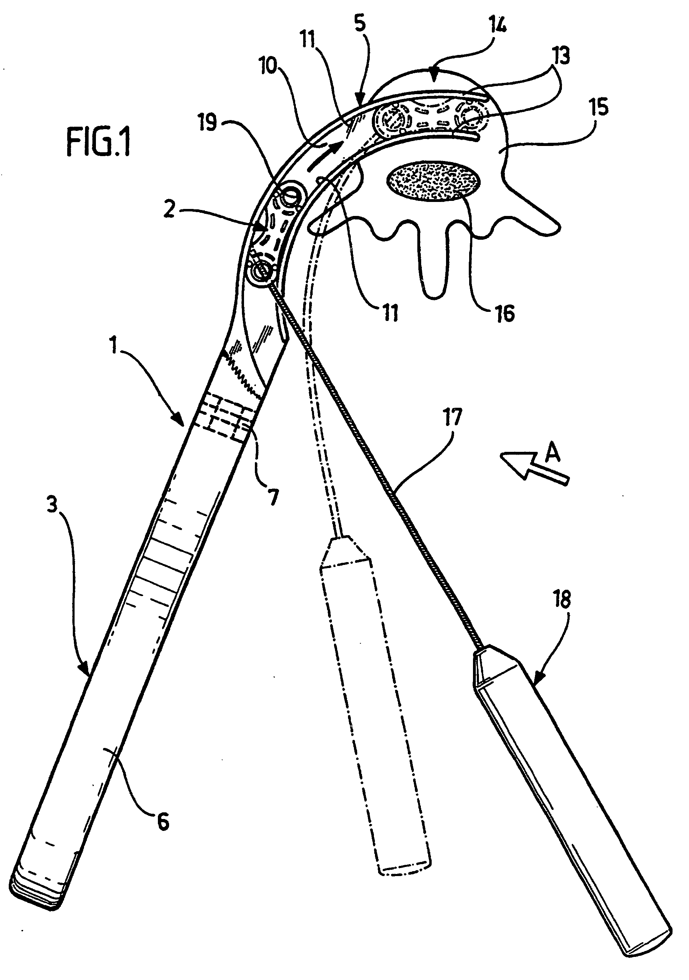

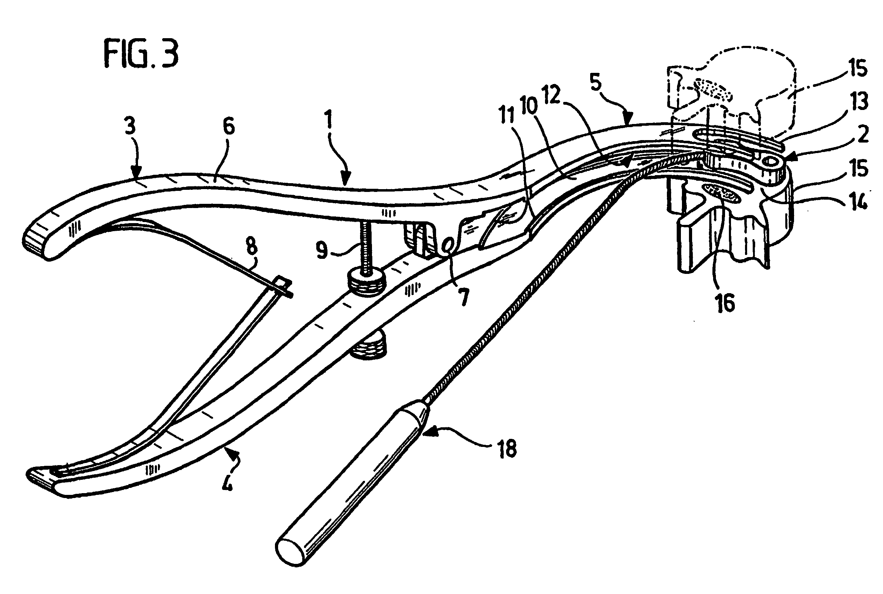

Das in den Figuren 1 bis 3 dargestellte Instrument 1 zum Einführen eines Zwischenwirbelimplantats 2 ist nach Art einer Zange ausgebildet und umfaßt zwei Arme 3, 4, die im wesentlichen gleich ausgebildet sind. Jeder Arm weist einen ebenen Führungskörper 5 und ein sich daran anschließendes, aus der Ebene des Führungskörpers 5 seitlich heraustretendes Griffteil 6 auf, beide Arme 3, 4 sind im Übergangsbereich zwischen den Führungskörpern 5 und den Griffteilen 6 durch ein Drehgelenk 7 derart schwenkbar miteinander verbunden, daß beim Gegeneinanderdrücken der Griffteile 6 die Führungskörper 5 auseinandergeschwenkt werden.

Zwischen den Griffteilen 6 sind Federelemente 8 angeordnet, die die Griffteile 6 auseinanderspreizen, ausserdem wird einer der Griffteile 6 von einer Spindel 9 durchsetzt, die mehr oder weniger tief in Richtung auf das jeweils andere Griffteil 6 eingedreht werden kann und einen Anschlag bildet, mit dem die Annäherung der beiden Griffteile 6 begrenzt werden kann.

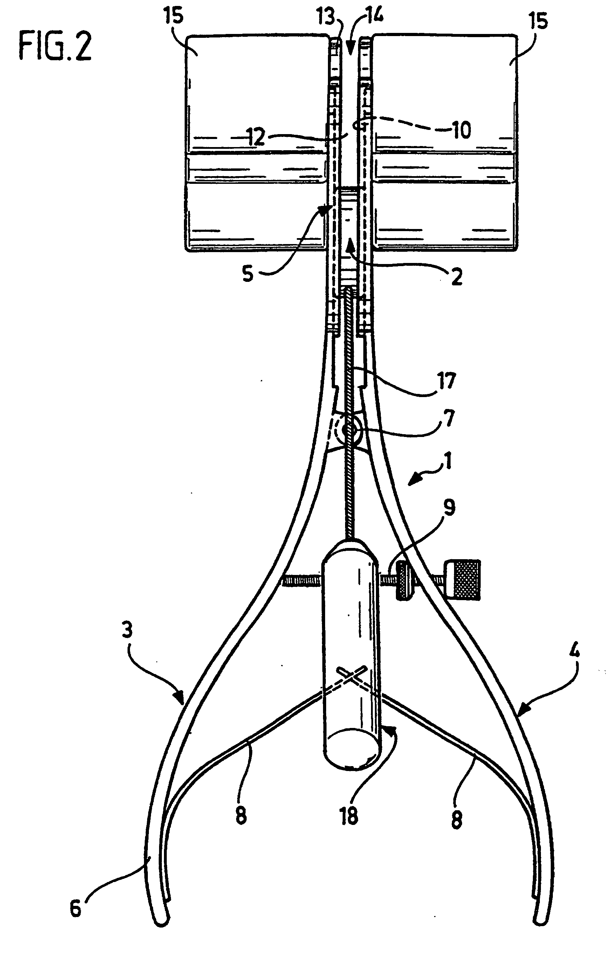

Die beiden Führungskörper 5 sind im Querschnitt U-förmig ausgebildet und weisen einander gegenüberliegende ebene Führungsfläche 10 und diese seitlich begrenzende, sich über die gesamte Länge der Führungsfläche 10 erstreckende, in Richtung auf den jeweils anderen Führungskörper vorstehende Seitenwände 11 auf, die auch bei einander maximal angenäherten Führungskörpern 5 einen schlitzförmigen Zwischenraum 12 zwischen sich einhalten (Figur 2). Die beiden Führungskörper 5 bilden somit zwischen sich eine Führungsbahn aus, die an der Oberseite und an der Unterseite durch die beiden Führungsflächen 10 begrenzt wird, an den Seiten durch die Seitenwände 11.

Die Führungskörper 5 sind in der durch die Führungsfläche 10 aufgespannten Ebene gebogen ausgebildet, beispielsweise erstreckt sich dieser Bogen über einen Winkel von 90°, so daß auch die Führungsbahn bogenförmig ist (Figur 1). Dabei kann die Führungsbahn an ihrem dem Drehgelenk 7 naheliegenden Ende seitlich aus den Führungskörpern 5 austreten, so daß an dieser Stelle ein Zwischenwirbelimplantat 2 in die Führungsbahn eingeschoben werden kann (Figur 1).

Am freien Ende der Führungsflächen 10 setzen sich die Seitenwände 11 in Verlängerung der Führungsflächen 10 weiter fort und bilden somit paarweise nebeneinander verlaufende Verlängerungen 13 aus, die auch als Zinken bezeichnet werden könnten.

Dadurch ergibt sich weiterhin eine seitliche Führung von längs der Führungsbahn vorgeschobenen Zwischenwirbelimplantaten, da die Führungsflächen 10 aber früher enden, fällt die Führung nach oben und unten weg, im Bereich zwischen den Verlängerungen 13 sind praktisch Fenster ausgebildet, durch die längs der Führungsbahn bis an deren Ende vorgeschobene Implantate nach oben und unten aus der Führungsbahn austreten können.

Zum Einsetzen eines Zwischenwirbelimplantats 2 wird das beschriebene Implantat 1 mit den Führungskörpern 5 durch einen Körperzugang bis in den Zwischenwirbelraum 14 zwischen zwei benachbarten Wirbelkörpern 15 eingeführt, und zwar derart, daß die zinkenförmigen Verlängerungen 13 seitlich in den Zwischenwirbelraum eintreten, und zwar unmittelbar angrenzend an den Wirbelkanal 16 und ventral von diesem. Die Führungsflächen 10 enden dabei kurz nach Eintritt in den Zwischenwirbelraum 14, während sich die zinkenförmigen Verlängerungen 13 vollständig in den Zwischenwirbelraum 14 hinein erstrecken.

Nach dem Einführen der Verlängerungen 13 in den Zwischenwirbelraum 14 werden die Führungskörper 5 mittels der Griffteile 6 auseinandergespreizt, so daß dadurch auch der Abstand der Wirbelkörper 15 vergrößert wird, das heißt der Zwischenwirbelraum 14 wird aufgeweitet.

Ein in den Zwischenwirbelraum 14 einzusetzendes Zwischenwirbelimplantat 2, das beispielsweise die Form einer länglichen Platte haben kann, wird dann längs der Führungsbahn, die durch die Führungskörper 5 ausgebildet wird, in den Zwischenwirbelraum 14 eingeschoben.

Zu diesem Zweck wird das plattenförmige Zwischenwirbelimplantat 2 mit dem dünnen flexiblen Schaft 17 eines Einsetzinstruments 18 verbunden, beispielsweise durch Einschrauben des flexiblen Schafts 17 in ein Innengewinde des Zwischenwirbelimplantats.

Mittels dieses Einsetzinstruments 18 wird das Zwischenwirbelimplantat 2 längs der Führungsbahn so weit verschoben, bis es sich im Zwischenwirbelraum 14 zwischen den zinkenförmigen Verlängerungen 13 befindet und damit das Ende der Führungsbahn erreicht. Das Vorschieben ist ohne weiteres möglich, da die benachbarten Wirbelkörper 15 durch die Führungskörper 5 in ausreichendem Abstand gehalten werden.

Sobald das Zwischenwirbelimplantat 2 seine Position zwischen den zinkenförmigen Verlängerungen 13 erreicht hat, fällt es aus der Führungsbahn, die durch die Führungskörper 5 gebildet wird, heraus und legt sich an die benachbarten Wirbelkörper an. Der Operateur kann jetzt den Druck auf die Griffteile 6 des Instruments 1 nachlassen und damit die Aufspreizung des Zwischenwirbelraums 14 beenden, so daß sich die Wirbelkörper 15 gegeneinander verschieben und beidseitig an dem Zwischenwirbelimplantat 2 zur Anlage kommen. Das Instrument 1 kann danach ohne weiteres wieder aus dem Zwischenwirbelraum 14 und aus dem Körper herausgezogen werden.

Beim Einschieben des Implantats ist es vorteilhaft, daß der Schaft 17 des Einsetzinstruments 18 nicht unbedingt längs der Führungsbahn angeordnet sein muß, sondern seitlich aus der Führungsbahn austreten kann, da der Schaft 17 durch den Zwischenraum 12 zwischen den Führungskörpern 5 hindurchtritt.

Sobald das Zwischenwirbelimplantat 2 in der beschriebenen Weise in den Zwischenwirbelraum 14 eingesetzt ist, kann auch das Einsetzinstrument 18 wieder entfernt werden, beispielsweise durch Herausdrehen des Schafts 17 aus dem Einschraubgewinde.

Durch entsprechende Einführkanäle im Zwischenwirbelimplantat 2 kann Knochenmaterial in den von dem Zwischenwirbelimplantat 2 nicht ausgefüllten Teil des Zwischenwirbelraums 14 und gegebenenfalls in Durchbrechungen 19 des Zwischenwirbelimplantats 2 eingefüllt werden. Dies kann mit Hilfe eines Einfüllinstruments 20 erfolgen, welches ein Einfüllrohr 21 umfaßt, in das Knochenmaterial eingefüllt werden kann. Mittels eines Stößels 22 kann das Knochenmaterial aus dem Einfüllrohr 21 an dessen freiem Ende herausgeschoben werden. Setzt man das freie Ende dieses Einfüllrohrs 21 an dem beschriebenen Einfüllkanal des Zwischenwirbelimplantats 2 an, so tritt Knochenmaterial durch diesen Einfüllkanal hindurch und gelangt in die Durchbrechungen 19 des Zwischenwirbelimplantats 2 und durch das gesamte Zwischenwirbelimplantat 2 hindurch in den ventral vom Zwischenwirbelimplantat 2 angeordneten Teil des Zwischenwirbelraums 14, so daß das Zwischenwirbelimplantat 2 sehr effektiv von Knochensubstanz umbettet wird.

Die Form des Zwischenwirbelimplantats 2 kann in diesem Falle recht verschieden sein. Bei den Ausführungsbeispielen der Figuren 1 bis 3 ist ein Zwischenwirbelimplantat 2 dargestellt, welches länglich und leicht gebogen ausgebildet ist, wobei es im mittleren Bereich eine geringere Breite hat als im Endbereich. In diesem mittleren Bereich ist das Implantat auf seiner ventralen Längsseite vertieft, so daß dort ein Aufnahmeraum für Knochenmaterial entsteht, der bei eingesetztem Zwischenwirbelimplantat 2 zu einer formschlüssigen Einbettung des Zwischenwirbelimplantats 2 im Zwischenwirbelraum 14 führt.

Ein ähnlich geformtes Implantat ohne eine derartige Vertiefung ist in Figur 5 dargestellt.

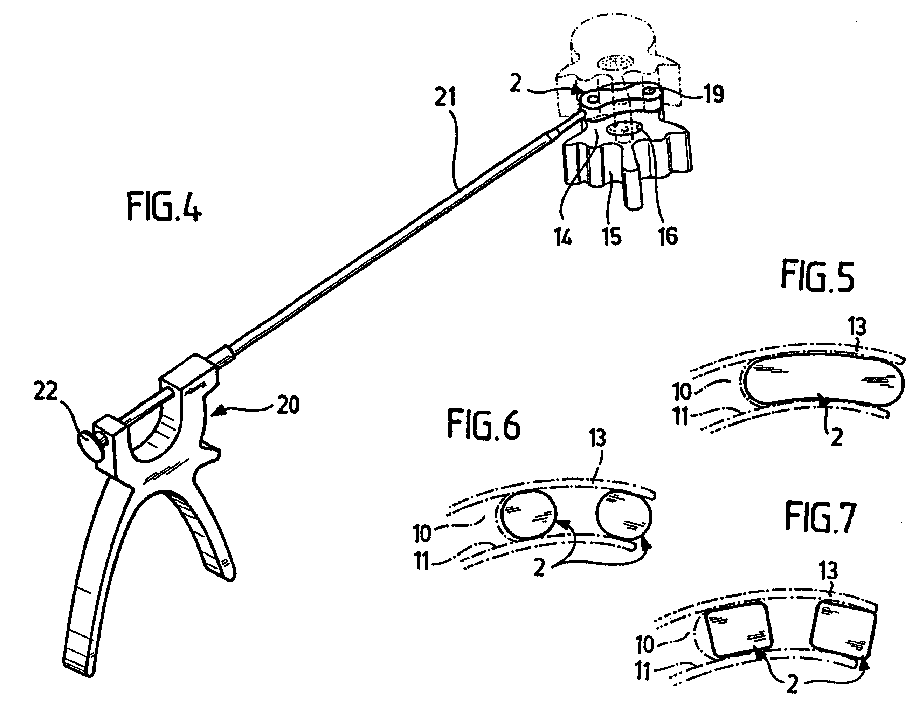

Im Ausführungsbeispiel der Figur 6 werden statt eines Zwischenwirbelimplantats 2 zwei Zwischenwirbelimplantate 2 im Abstand zueinander eingesetzt, dieses Einsetzen erfolgt in nacheinander durchgeführten Einsetzvorgängen genau in derselben Weise, wie dies anhand des Implantats der Figuren 1 bis 3 beschrieben worden ist. Die Implantate 2 der Figur 6 haben eine im wesentlichen ovalen oder elliptischen Querschnitt, beim Ausführungsbeispiel der Figur 7 sind zwei Implantate mit im wesentlichen rechteckigem Querschnitt vorgesehen, auch diese werden in ähnlicher Weise eingesetzt.

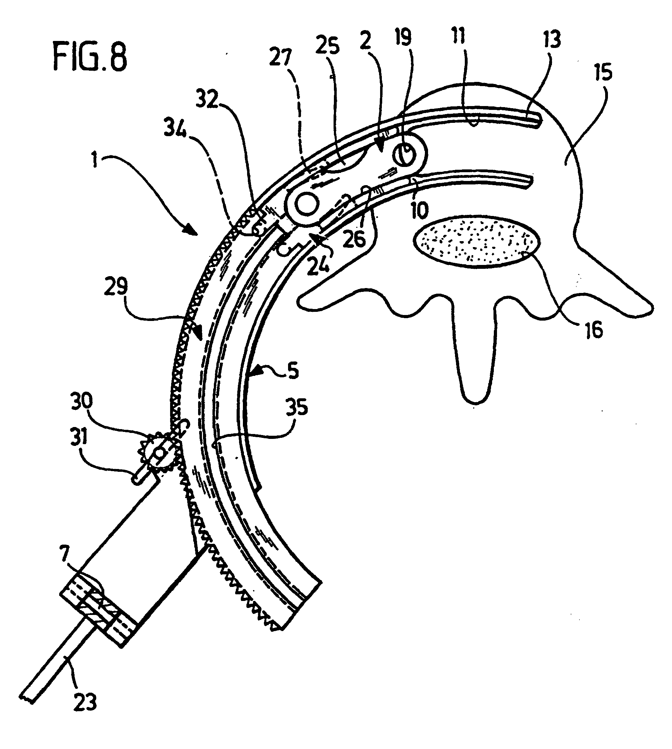

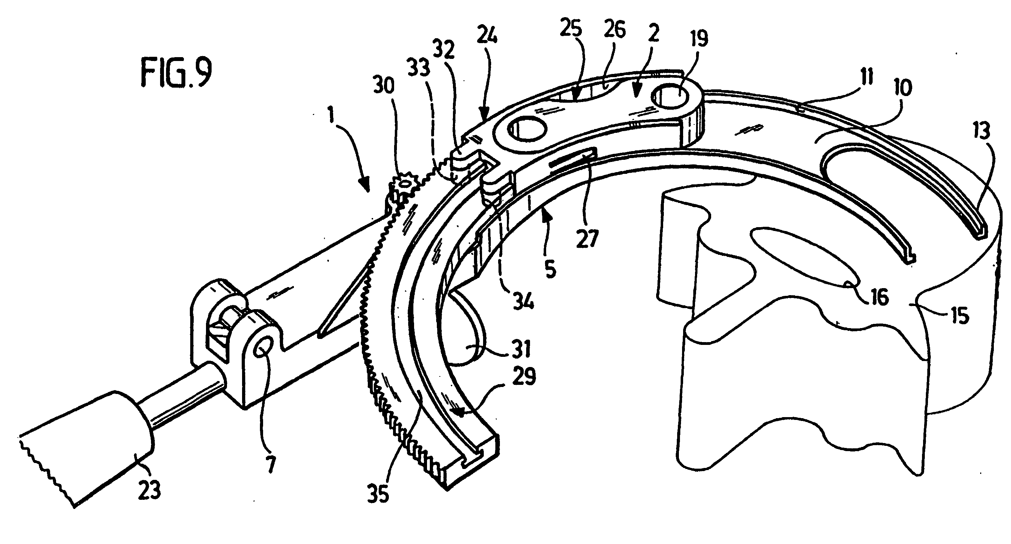

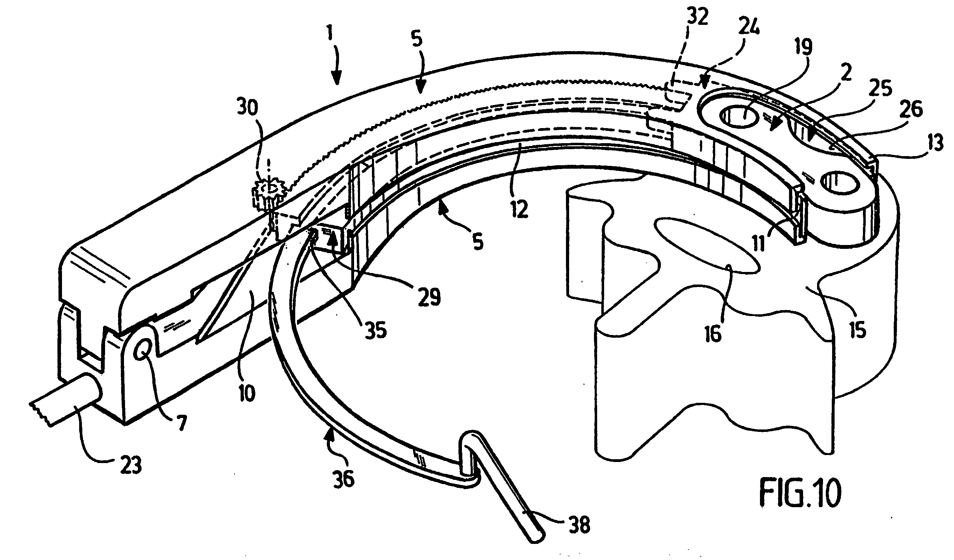

In den Figuren 8 bis 11 ist ein weiteres bevorzugtes Ausführungsbeispiel eines Instrumentes 1 dargestellt, das ähnlich aufgebaut ist wie das Instrument der Figuren 1 bis 3, einander entsprechende Teile tragen daher dieselben Bezugszeichen.

Im Unterschied zu dem Ausführungsbeispiel der Figuren 1 bis 3 sind die Führungskörper 5 bei diesem Ausführungsbeispiel nicht mit branchenartigen Griffteilen 6 versehen, das Instrument hat also nicht die Gestalt einer Zange. Die beiden Führungskörper 5 sind vielmehr ebenso über ein Drehgelenk 7 miteinander verbunden und enden an diesem Drehgelenk 7, einer der beiden Führungskörper 5 trägt dabei einen Haltegriff 23. Alternativ könnte auch vorgesehen sein, eines der beiden branchenartigen Griffteile 6 im Bereich des Gelenkes 7 abnehmbar auszugestalten und das verbleibende Griffteil als Haltegriff zu verwenden.

Bei einer solchen Ausbildung des Instrumentes erfolgt das Aufspreizen nicht durch das Zusammendrücken von branchenartigen Griffteilen, sondern allein dadurch, daß die längs der Führungsbahn vorgeschobenen Teile beim Vorschieben die Führungskörper 5 auseinanderdrükken und dabei mit deren freien Enden, die zwischen die Wirbelkörper 15 eingreifen, den Abstand zwischen den Wirbelkörpern 15 vergrößern. Dazu sind die Führungskörper 5 derart ausgebildet, daß sie an der Einschubseite einen Abstand voneinander aufweisen, der größer ist als die Höhe der zwischen den Führungskörpern 5 vorgeschobenen Teile, am Austrittsende der Führungskörper 5 jedoch einen kleineren Abstand. Dadurch können die ungespreizten Führungskörper 5 mit ihrem freien Ende in den unaufgeweiteten Zwischenwirbelraum eintreten, die zwischen den Führungskörpern vorgeschobenen Teile können ohne weiteres an der Einschubseite eingeschoben werden, und erst durch die Vorschubbewegung der vorgeschobenen Teile selbst erfolgt das Aufspreizen der Führungskörper 5 und damit das Aufweiten des Zwischenwirbelraumes 14.

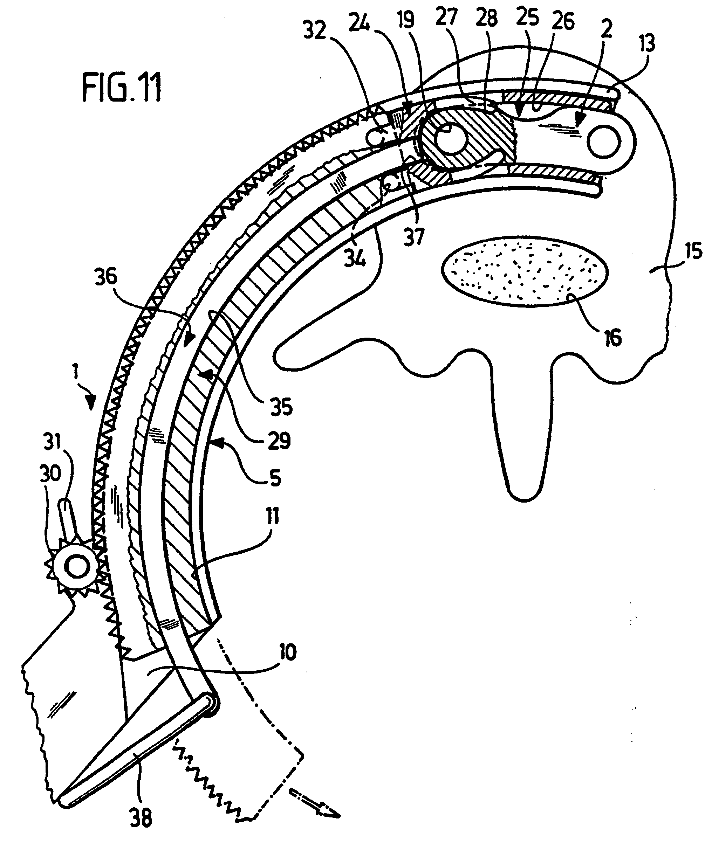

Die zwischen den Führungskörpern 5 vorgeschobenen Teile können ganz einfach durch das Zwischenwirbelimplantat 2 selbst gebildet werden, es ist aber vorteilhaft, wenn - wie im Ausführungsbeispiel der Figuren 8 bis 11 dargestellt - zwischen den Führungskörpern 5 ein spezieller Vorschubkörper 24 längs der Führungsbahn verschiebbar gelagert ist, der an den beiden Führungskörpern 5 anliegt und diese Aufspreizung vornimmt. Der Vorschubkörper 24 ist länglich ausgebildet und liegt beidseitig an den Seitenwänden 11 der Führungskörper 5 an, so daß er längs der Führungsbahn exakt geführt wird. Er weist einen länglichen Aufnahmeraum 25 auf, der durch die im wesentlichen parallel zueinander verlaufenden Seitenwände 26 des Vorschubkörpers 24 gebildet wird und an der Oberseite und an der Unterseite sowie an der in Vorschubrichtung vorn liegenden Stirnseite offen ist. In diesen Aufnahmeraum 25 wird von der offenen Seite her das Zwischenwirbelimplantat 2 zwischen die Seitenwände 26 eingeschoben und in der eingeschobenen Stellung durch elastische Rastzungen 27 gehalten, die in die Seitenwände 26 des Vorschubkörpers 24 eingearbeitet sind und die elastisch in seitliche Rücksprünge 28 des Zwischenwirbelimplantates 2 eingreifen (Figur 11).

In dem in der Zeichnung dargestellten Ausführungsbeispiel ist die Höhe des Zwischenwirbelimplantates 2 gleich der Höhe des Vorschubkörpers 24, in diesem Falle spreizen Zwischenwirbelimplantat und Vorschubkörper gemeinsam die Führungskörper 5 auf, wenn sie längs der Führungsbahn vorgeschoben werden. Das Zwischenwirbelimplantat könnte auch eine etwas kleinere Höhe aufweisen als der Vorschubkörper, in diesem Falle würde die Aufspreizung ausschließlich durch den Vorschubkörper erfolgen.

Der Vorschubkörper 24 ist verbunden mit einer gebogenen Zahnstange 29, die in einem der beiden Führungskörper 5 geführt ist und die mit einem Zahnrad 30 kämmt, das an einem der Führungskörper 5 drehbar gelagert ist und über einen Drehgriff 31 verdrehbar ist, so daß die Zahnstange 29 längs der Führungsbahn vor- und zurückgeschoben werden kann. Die Verbindung zwischen Vorschubkörper 24 und Zahnstange 29 ist dabei lösbar, im dargestellten Ausführungsbeispiel trägt der Vorschubkörper 24 an zwei vorstehenden Lappen 32 je einen senkrecht abstehenden Stift 33, der in eine Bohrung 34 am Ende der Zahnstange 29 einsteckbar ist.

Auf der Oberseite der Zahnstange 29 ist eine nach oben offene Längsmittelnut 35 angeordnet, in der ein gebogenes, stabförmiges Rückhalteelement 36 vorschiebbar ist. Das vordere Ende der Längsmittelnut 35 steht mit einer Öffnung 37 im Vorschubkörper 24 in Verbindung, durch diese Öffnung 37 kann das Rückhalteelement 36 in den Aufnahmeraum 25 vorgeschoben werden und sich dort an das im Aufnahmeraum 25 gehaltene Zwischenwirbelimplantat 2 anlegen (Figur 11).

Zum Einsetzen des Zwischenwirbelimplantates 2 wird dieses in den Aufnahmeraum 25 eingeschoben, bis die Rastzungen 27 in die Rücksprünge 28 einschnappen. Der Vorschubkörper 24 kann dann an der Einschubseite zwischen die Führungskörper 5 eingeschoben und durch Einstecken der Stifte 33 in die Bohrungen 34 mit der Zahnstange 29 verbunden werden, die ebenfalls zwischen die Führungskörper teilweise eingeschoben wird.

Dieses Einschieben des Vorschubkörpers 24 und der Zahnstange 29 kann bereits vor dem Einsetzen des Instrumentes in den Körper erfolgen, vorteilhaft wird es aber sein, zunächst das leere Instrument 1 in den Körper einzuführen, bis das freie Ende der Führungskörper in den Zwischenwirbelraum 14 eintritt, in den das Zwischenwirbelimplantat 2 eingeschoben werden soll.

Durch Verdrehung des Zahnrades 30 wird die Zahnstange 29 zwischen den Führungskörpern vorgeschoben und schiebt dadurch den Vorschubkörper 24 mit dem Zwischenwirbelimplantat 2 allmählich bis in den Zwischenwirbelraum 14 vor, der durch Spreizen der Führungskörper 5 bei dieser Vorschubbewegung aufgeweitet wird.

Sobald sich das Zwischenwirbelimplantat 2 in der Endposition im Zwischenwirbelraum 14 befindet, wird das Rückhalteelement 36 eingeschoben, bis es am Zwischenwirbelimplantat 2 zur Anlage kommt. Die Einschubtiefe des Rückhalteelementes 36 kann dabei durch einen Anschlag begrenzt werden, der beispielsweise durch eine seitliche Abbiegung 38 des Rückhalteelementes 36 gebildet wird, die am Führungskörper 5 anschlägt (Figur 11). Beim Zurückziehen des Vorschubkörpers 24 und der Zahnstange 29 wird die elastische Rastverbindung zwischen den Rastzungen 27 und den Rücksprüngen 28 auf diese Weise gelöst, da das Zwischenwirbelimplantat 2 durch das Rückhalteelement 36 im Zwischenwirbelraum 14 zurückgehalten wird. Zu diesem Zweck ist das Rückhalteelement 36 über in der Zeichnung nicht dargestellte Mittel am Führungskörper 5 lösbar fixierbar. Auf diese Weise können Vorschubkörper 24, Zahnstange 29 und anschließend auch das Rückhalteelement 36 aus dem Instrument 1 herausgezogen werden, wobei die Aufweitung der den Zwischenwirbelraum 14 begrenzenden Wirbelkörper 15 aufgehoben wird, die Wirbelkörper 15 stützen sich nunmehr auf dem Zwischenwirbelimplantat 2 ab, und danach kann das Instrument ganz aus dem Zwischenwirbelraum 14 herausgezogen werden.

## Patentansprüche

1. Chirurgisches Instrument zum Einführen von Zwischenwirbelimplantaten (2) in den Zwischenwirbelraum (14) zwischen benachbarten Wirbelkörpern (15) mit zwei einander gegenüberliegenden Führungskörpern (5) mit einer zum jeweils anderen Führungskörper (5) gerichteten Führung (10, 11), die gemeinsam eine Führungsbahn zwischen sich ausbilden, längs der ein Zwischenwirbelimplantat (2) seitlich in den Zwischenwirbelraum (14) einschiebbar ist, **dadurch gekennzeichnet, daß** die Führungskörper (5) in ihrem gegenseitigen Abstand verstellbar sind.

2. Chirurgisches Instrument nach Anspruch 1, **dadurch gekennzeichnet, daß** die Führungsbahn in der Verschiebeebene bogenförmig verläuft.

3. Chirurgisches Instrument nach einem der voranstehenden Ansprüche, **dadurch gekennzeichnet, daß** der Zwischenraum zwischen den Führungskörpern (5) zumindest einseitig längs der Führungsbahn offen ist.

4. Chirurgisches Instrument nach einem der voranstehenden Ansprüche, **dadurch gekennzeichnet, daß** die Führungskörper (5) ein freies Ende aufweisen und dort Verlängerungen (13) tragen, die an den Zwischenwirbelraum (14) bildenden Wirbelkörpern (15) anlegbar sind und die so neben der Führungsbahn angeordnet sind, daß das längs der Führungsbahn vorgeschobene Zwischenwirbelimplantat (2) am Ende der Führungsbahn neben der Verlängerung (13) an die benachbarten Wirbelkörper (15) anlegbar ist.

5. Chirurgisches Instrument nach Anspruch 4, **dadurch gekennzeichnet, daß** die Verlängerungen (13) als paarweise in Verschieberichtung vom Ende der Führungsbahn in dessen Fortsetzung vorstehende Zinken ausgebildet sind.

6. Chirurgisches Instrument nach einem der voranstehenden Ansprüche, **dadurch gekennzeichnet, daß** die Führungsbahn an der Oberseite und an der Unterseite durch zwei Führungsflächen (10) begrenzt wird und an den Seiten durch Seitenwände (11), die einen schlitzförmigen Zwischenraum (12) zwischen sich einhalten, so daß ein zwischen den Führungskörpern (5) geführtes Zwischenwirbelimplantat (2) in der Führungsbahn auch dann geführt ist, wenn der Abstand der Führungskörper (50) sich vergrößert.

7. Chirurgisches Instrument nach einem der voranstehenden Ansprüche, **dadurch gekennzeichnet, daß** die Führungskörper (5) ein freies Ende aufweisen und an dem dem freien Ende gegenüberliegenden Ende (7) schwenkbar miteinander verbunden sind.

8. Chirurgisches Instrument nach einem der voranstehenden Ansprüche, **dadurch gekennzeichnet, daß** jeder Führungskörper (5) eine ebene Anlagefläche (10) für das Zwischenwirbelimplantat (2) und diese seitlich begrenzende, parallel zueinander längs des Führungskörpers (5) verlaufende Führungswände (11) aufweist, die in Richtung auf den anderen Führungskörper (5) über die Anlagefläche (10) vorstehen.

9. Chirurgisches Instrument nach einem der voranstehenden Ansprüche, **dadurch gekennzeichnet, daß** die Führungskörper (5) ein freies Ende aufweisen und daß die Führungsbahn an ihrem dem freien Ende der Führungskörper (5) gegenüberliegenden Ende seitlich aus dem Instrument austritt, so daß dort ein Zwischenwirbelimplantat (2) in die Führungsbahn einschiebbar ist.

10. Chirurgisches Instrument nach einem der Ansprüche 7 bis 9, **dadurch gekennzeichnet, daß** beide Führungskörper (5) mit Griffbranchen (6) fest verbunden sind, die sich über die gelenkige Verbindungsstelle (7) der Führungskörper (5) hinaus erstrecken.

11. Chirurgisches Instrument nach einem der voranstehenden Ansprüche, **dadurch gekennzeichnet, daß** an ihm ein Anschlag (9) zur Einhaltung eines Mindestabstands zwischen den beiden Führungskörpern (5) angeordnet ist.

12. Chirurgisches Instrument nach Anspruch 11, **dadurch gekennzeichnet, daß** der Anschlag (9) verstellbar ist.

13. Chirurgisches Instrument nach einem der voranstehenden Ansprüche, **dadurch gekennzeichnet, daß** zwischen den beiden Führungskörpern (5) längs der von diesen gebildeten Führungsbahn ein Vorschubkörper (24) verschiebbar gelagert ist, der eine lösbare Halterung für das Zwischenwirbalimplantat (2) aufweist.

14. Chirurgisches Instrument nach Anspruch 13, **dadurch gekennzeichnet, daß** der Vorschubkörper (24) einen zu seinem in Vorschubrichtung vorderen Ende hin offenen Aufnahmeraum (25) für das Zwischenwirbelimplantat (2) aufweist.

15. Chirurgisches Instrument nach Anspruch 14, **dadurch gekennzeichnet, daß** der Aufnahmeraum (25) durch zwei längs der Führungsbahn verlaufende Seitenwände (26) begrenzt wird.

16. Chirurgisches Instrument nach einem der Ansprüche 13 bis 15, **dadurch gekennzeichnet, daß** die lösbare Halterung elastische Rasten (27) umfaßt, die in Rücksprünge d(28) einfedern.

17. Chirurgisches Instrument nach einem der Ansprüche 14 bis 16, **dadurch gekennzeichnet, daß** der Vorschubkörper (24) an seinem in Vorschubrichtung hinteren Ende eine Zugangsöffnung (37) für ein an dem Zwischenwirbelimplantat (2) anlegbares Rückhalteelement (36) aufweist.

18. Chirurgisches Instrument nach Anspruch 17, **dadurch gekennzeichnet, daß** das Rückhalteelement (36) entsprechend der Führungsbahn gebogen ist.

19. Chirurgisches Instrument nach einem der Ansprüche 13 bis 18, **dadurch gekennzeichnet, daß** der Vorschubkörper (24) mit einer längs der Führungsbahn verschiebbaren, gebogenen Vorschubstange (29) verbunden ist.

20. Chirurgisches Instrument nach Anspruch 19, **dadurch gekennzeichnet, daß** die Verbindung (33, 34) zwischen Vorschubkörper (24) und Vorschubstange (29) lösbar ist.

21. Chirurgisches Instrument nach Anspruch 19 oder 20, **dadurch gekennzeichnet, daß** die Vorschubstange (29) als Zahnstange ausgebildet ist, welche mit einem an einem Führungskörper (5) drehbar gelagerten Zahnrad (30) kämmt.

22. Chirurgisches Instrument nach einem der Ansprüche 19 bis 21, **dadurch gekennzeichnet, daß** die Vorschubstange (29) eine Längsnut (35) zur Aufnahme und Führung eines Rückhalteelementes (36) für das Zwischenwirbelimplantat aufweist.

23. Chirurgisches Instrument nach einem der Ansprüche 7 bis 22 und durch dieses in einen Zwischenwirbelraum einschiebbares Zwischenwirbelimplantat (2), **dadurch gekennzeichnet, daß** die Führungskörper (5) von ihrer Schwenklagerstelle (7) bis zu ihrem freien Ende hin einen abnehmenden Abstand zueinander aufweisen, der an der in Vorschubrichtung hinten angeordneten Einschubseite der Führungsbahn größer ist als die Höhe des Zwischenwirbelimplantates und gegebenenfalls des Vorschubkörpers (24), an der in Vorschubrichtung vorne liegenden Austrittsseite der Führungsbahn jedoch kleiner als die Höhe des Zwischenwirbelimplantates und/oder des Vorschubkörpers (24).

24. Chirurgisches Instrument nach Anspruch 23, **dadurch gekennzeichnet, daß** der Vorschubkörper (24) und/oder die Führungskörper (5) an den aneinander anliegenden Flächen aus einem reibungsarmen Material bestehen.

## Claims

1. A surgical instrument for inserting intervertebral implants (2) into the intervertebral space (14) between adjacent vertebrae (15), comprising two opposing guide members (5) each having a guide (10, 11) facing the respective other guide member (5), the guides (10, 11) together forming a guide path between them, along which an intervertebral implant (2) is laterally pushable into the intervertebral space (14), **characterised in that** the distance between the guide members (5) is adjustable.

2. A surgical instrument according to claim 1, **characterised in that** the guide path is curved in the displacement plane.

3. A surgical instrument according to either one of the preceding claims, **characterised in that** the gap between the guide members (5) is open along the guide path at least on one side.

4. A surgical instrument according to any one of the preceding claims, **characterised in that** the guide members (5) have a free end carrying extensions (13) which are placeable against vertebrae (15) forming the intervertebral space (14) and are arranged next to the guide path so that the intervertebral implant (2), which has been pushed along the guide path, is placeable against the adjacent vertebrae (15) next to the extension (13) at the end of the guide path.

5. A surgical instrument according to claim 4, **characterised in that** the extensions (13) are formed as prongs projecting in pairs from the end of the guide path as a continuation thereof in the displacement direction.

6. A surgical instrument according to any one of the preceding claims, **characterised in that** the guide path is bounded at the top and bottom by two guide surfaces (10) and at the sides by side walls (11), leaving a slot-type gap (12) between them so that an intervertebral implant (2) guided between the guide members (5) is guided in the guide path even when the distance between the guide members (5) increases.

7. A surgical instrument according to any one of the preceding claims, **characterised in that** the guide members (5) have a free end and are pivotably connected to one another at the end (7) opposite the free end.

8. A surgical instrument according to any one of the preceding claims, **characterised in that** each guide member (5) has a flat contact surface (10) for the intervertebral implant (2) and guide walls (11) laterally bounding the contact surface (10) and extending parallel to one another along the guide member (5), the guide walls (11) projecting beyond the contact surface (10) towards the other guide member (5).

9. A surgical instrument according to any one of the preceding claims, **characterised in that** the guide members (5) have a free end and **in that** the guide path issues laterally from the instrument at its end opposite the free end of the guide members (5) so that an intervertebral implant (2) is insertable into the guide path at that point.

10. A surgical instrument according to any one of claims 7 to 9, **characterised in that** the two guide members (5) are fixedly connected to gripping arms (6) extending beyond the articulated connection point (7) of the guide members (5).

11. A surgical instrument according to any one of the preceding claims, **characterised in that** a stop (9) for maintaining a minimum distance between the two guide members (5) is arranged on the surgical instrument.

12. A surgical instrument according to claim 11, **characterised in that** the stop (9) is adjustable.

13. A surgical instrument according to any one of the preceding claims, **characterised in that** a pushing member (24) is mounted between the two guide members (5) so as to be displaceable along the guide path formed by the latter and has a releasable holder for the intervertebral implant (2).

14. A surgical instrument according to claim 13, **characterised in that** the pushing member (24) has a holding space (25) provided for the intervertebral implant (2) and open towards its front end in the pushing direction.

15. A surgical instrument according to claim 14, **characterised in that** the holding space (25) is bounded by two side walls (26) extending along the guide path.

16. A surgical instrument according to any one of claims 13 to 15, **characterised in that** the releasable holder comprises resilient detents (27) which spring into recesses (28).

17. A surgical instrument according to any one of claims 14 to 16, **characterised in that** the pushing member (24) has, at its rear end in the pushing direction, an access opening (37) for a retaining element (36) placeable against the intervertebral implant (2).

18. A surgical instrument according to claim 17, **characterised in that** the retaining element (36) is curved in conformity with the guide path.

19. A surgical instrument according to any one of claims 13 to 18, **characterised in that** the pushing member (24) is connected to a curved push rod (29) displaceable along the guide path.

20. A surgical instrument according to claim 19, **characterised in that** the connection (33, 34) between the pushing member (24) and the push rod (29) is disengageable.

21. A surgical instrument according to claim 19 or 20, **characterised in that** the push rod (29) is formed as a rack meshing with a pinion (30) rotatably mounted on a guide member (5).

22. A surgical instrument according to any one of claims 19 to 21, **characterised in that** the push rod (29) has a longitudinal groove (35) for receiving and guiding a retaining element (36) for the intervertebral implant.

23. A surgical instrument according to any one of claims 7 to 22 and an intervertebral implant (2) insertable into an intervertebral space by means of this surgical instrument, **characterised in that** the distance between the guide members (5) decreases from their pivot point (7) towards their free end, this distance being greater than the height of the intervertebral implant and, if applicable, the pushing member (24) at the insertion end of the guide path, arranged at the rear in the pushing direction, but smaller than the height of the intervertebral implant and/or the pushing member (24) at the exit end of the guide path, located at the front in the pushing direction.

24. A surgical instrument according to claim 23, **characterised in that** the contacting surfaces of the pushing member (24) and/or the guide members (5) consist of a low-friction material,

## Revendications

1. Instrument chirurgical pour l'introduction d'implants intervertébraux (2) dans l'espace intervertébral (14) situé entre les corps de vertèbres adjacents (15) avec deux corps de guidage (5) opposés l'un à l'autre avec un guidage (10, 11) orienté vers chaque autre corps de guidage (5) qui forment ensemble une glissière le long de laquelle un implant intervertébral (2) peut être glissé latéralement dans l'espace intervertébral (14), **caractérisé en ce que** les corps de guidage (5) sont réglables au niveau de leur distance réciproque.

2. Instrument chirurgical selon la revendication 1, **caractérisé en ce que** la glissière s'étend en forme d'arc dans le plan de déplacement.

3. Instrument chirurgical selon l'une des revendications précédentes, **caractérisé en ce que** l'espace intermédiaire situé entre les corps de guidage (5) est ouvert au moins de façon unilatérale le long de la glissière.

4. Instrument chirurgical selon l'une des revendications précédentes, **caractérisé en ce que** les corps de guidage (5) comportent une extrémité libre où ils supportent des prolongements (13) qui peuvent être posés contre les corps de vertèbres (15) formant l'espace intervertébral (14) et qui sont disposés à proximité de la glissière, de sorte que l'implant intervertébral (2) déplacé le long de la glissière peut être posé contre les corps de vertèbres adjacents (15) à l'extrémité de la glissière à côté du prolongement (13).

5. Instrument chirurgical selon la revendication 4, **caractérisé en ce que** les prolongements (13) sont configurés comme des pointes saillantes jumelées dans le sens de déplacement de l'extrémité de la glissière dans la continuation de celle-ci.

6. Instrument chirurgical selon l'une des revendications précédentes, **caractérisé en ce que** la glissière est limitée sur la face supérieure et sur la face inférieure par deux surfaces de guidage (10) et sur les côtés par des parois latérales (11) qui respectent entre elles un espace intermédiaire (12) en forme de fente, de sorte qu'un implant intervertébral (2) conduit entre les corps de guidage (5) est dirigé également dans la glissière lorsque la distance des corps de guidage (5) augmente.

7. Instrument chirurgical selon l'une des revendications précédentes, **caractérisé en ce que** les corps de guidage (5) comportent une extrémité libre et sont reliés les uns aux autres de façon orientable sur l'extrémité (7) opposée à l'extrémité libre.

8. Instrument chirurgical selon l'une des revendications précédentes, **caractérisé en ce que** chaque corps de guidage (5) comprend une surface de pose plane (10) pour l'implant intervertébral (2) et ces parois (11) à délimitation latérale et à développement parallèle les unes par rapport aux autres le long du corps de guidage (5) et qui font saillie sur la surface de pose (10) dans le sens des autres corps de guidage (5).

9. Instrument chirurgical selon l'une des revendications précédentes, **caractérisé en ce que** les corps de guidage (5) comportent une extrémité libre, et **en ce que** la glissière ressort latéralement de l'instrument sur son extrémité opposée à l'extrémité libre des corps de guidage (5), de sorte qu'un implant intervertébral (2) peut être introduit dans la glissière.

10. Instrument chirurgical selon l'une des revendications 7 à 9, **caractérisé en ce que** les deux corps de guidage (5) sont fermement reliés entre eux par des branches (6) qui s'étendent au-delà du point de jonction articulé (7) des corps de guidage (5).

11. Instrument chirurgical selon l'une des revendications précédentes, **caractérisé en ce qu'**une butée (9) est disposée sur celui-ci afin de respecter une distance minimale entre les deux corps de guidage (5).

12. Instrument chirurgical selon la revendication 11, **caractérisé en ce que** la butée (9) est réglable.

13. Instrument chirurgical selon l'une des revendications précédentes, **caractérisé en ce qu'**un corps d'avance (24) est logé et peut être déplacé entre les deux corps de guidage (5) le long de la glissière formée par ceux-ci, et comprend une fixation amovible pour l'implant intervertébral (2).

14. Instrument chirurgical selon la revendication 13, **caractérisé en ce que** le corps d'avance (24) comprend un espace de logement (25) pour l'implant intervertébral (2) ouvert sur son extrémité avant dans le sens d'avance.

15. Instrument chirurgical selon la revendication 14, **caractérisé en ce que** l'espace de logement (25) est délimité par deux parois latérales (26) s'étendant le long de la glissière.

16. Instrument chirurgical selon l'une des revendications 13 à 15, **caractérisé en ce que** la fixation amovible comprend des taquets d'enclenchement élastiques (27) qui font ressort dans des retours d(28).

17. Instrument chirurgical selon l'une des revendications 14 à 16, **caractérisé en ce que** le corps d'avance (24) comprend sur son extrémité arrière dans le sens d'avance un orifice d'accès (37) pour un élément de retenue (36) pouvant être posé contre l'implant intervertébral (2).

18. Instrument chirurgical selon la revendication 17, **caractérisé en ce que** l'élément de retenue (36) est incurvé conformément à la glissière.

19. Instrument chirurgical selon l'une des revendications 13 à 18, **caractérisé en ce que** le corps d'avance (24) est relié à un poussoir (29) incurvé pouvant être déplacé le long de la glissière.

20. Instrument chirurgical selon la revendication 19, **caractérisé en ce que** la liaison (33, 34) entre les corps d'avance (24) et le poussoir (29) est amovible.

21. Instrument chirurgical selon la revendication 19 ou 20, **caractérisé en ce que** le poussoir (29) est configuré comme une crémaillère qui actionne la roue dentée (30) logée de façon rotative sur un corps de guidage (5).

22. Instrument chirurgical selon l'une des revendications 19 à 21, **caractérisé en ce que** le poussoir (29) comprend une rainure longitudinale (35) pour le logement et le guidage d'un élément de retenue (36) pour l'implant intervertébral.

23. Instrument chirurgical selon l'une des revendications 7 à 22 et par cet implant intervertébral (2) pouvant être introduit dans un espace intervertébral, **caractérisé en ce que** les corps de guidage (5) présentent, de leur point d'appui pivotant (7) à leur extrémité libre, une distance décroissante l'un à l'autre qui est, sur la face insérable de la glissière disposée à l'arrière dans le sens d'avance, supérieure à la hauteur de l'implant intervertébral et éventuellement du corps d'avance (24), et qui est cependant inférieure, sur la face de sortie de la glissière située à l'avant dans le sens d'avance, à la hauteur de l'implant intervertébral et/ou du corps d'avance (24).

24. Instrument chirurgical selon la revendication 23, **caractérisé en ce que** le corps d'avance (24) et/ou les corps de guidage (5) sont composés d'un matériau présentant une faible friction sur les surfaces adjacentes.
